# EUROPEAN PATENT SPECIFICATION

(11) **EP 2 324 112 B1**
(45) Date of publication and mention of the grant of the patent: **30.05.2012**
(21) Application number: 09783067.3
(22) Date of filing: 16.09.2009
(51) Int. Cl.: C12N 7/00, A61K 39/12, C07K 14/08

(54) **MUTANT PESTIVIRUS WITH MUTATIONS IN CORE GENE AND NS3 REGION**
MUTIERTES PESTIVIRUS MIT MUTATIONEN IM CORE-GEN UND NS3-BEREICH
PESTIVIRUS MUTANT AVEC DES MUTATIONS DANS LE GÈNE CENTRAL ET LA RÉGION NS3

(30) Priority: 17.09.2008 EP 08164496
(43) Date of publication of application: 25.05.2011
(73) Proprietor: Intervet International B.V., 5831 AN Boxmeer (NL)
(72) Inventor: THIEL, Heinz-Jurgen, 35392 Giessen (DE); RUEMENAPF, Hans Tillmann, 35392 Giessen (DE); HEIMANN, Manuela, 35392 Giessen (DE)
(74) Representative: Keus, Jacobus Albertus Ronald
(86) International application number: PCT/EP2009/061989
(87) International publication number: WO 2010/031783

(56) References cited:
- US-B1- 7 332 170
- MAURER R ET AL: "Oronasal vaccination with classical swine fever virus (CSFV) replicon particles with either partial or complete deletion of the E2 gene induces partial protection against lethal challenge with highly virulent CSFV" VACCINE, BUTTERWORTH SCIENTIFIC. GUILDFORD, GB, vol. 23, no. 25, 9 May 2005 (2005-05-09), pages 3318-3328, XP004869064 ISSN: 0264-410X cited in the application
- FREY CAROLINE F ET AL: "Classical swine fever virus replicon particles lacking the Erns gene: a potential marker vaccine for intradermal application" VETERINARY RESEARCH 2006 SEP-OCT,, vol. 37, no. 5, 1 September 2006 (2006-09-01), pages 655-670, XP002504774
- MOSER C. ET AL.: "Cytopathogenic and noncytopathogenic RNA replicons of Classical Swine Fever Virus" J. VIROLOGY, vol. 73, no. 9, 1999, pages 7787-7794, XP002514786 cited in the application
- GU B ET AL: "The RNA helicase and nucleotide triphosphatase activities of the bovine viral diarrhea virus NS3 protein are essential for viral replication" JOURNAL OF VIROLOGY, THE AMERICAN SOCIETY FOR MICROBIOLOGY, US, vol. 74, no. 4, 1 February 2000 (2000-02-01), pages 1794-1800, XP002474038 ISSN: 0022-538X
- GRASSMANN C W ET AL: "Assignment of the multifunctional NS3 protein of bovine viral diarrhea virus during RNA replication: an in vivo and in vitro study" JOURNAL OF VIROLOGY, THE AMERICAN SOCIETY FOR MICROBIOLOGY, US, vol. 73, no. 11, 1 November 1999 (1999-11-01), pages 9196-9205, XP002474037 ISSN: 0022-538X
- XU J. VIROL. vol. 71, no. 7, 1997, pages 5312 - 5322

## Description

The present invention relates to mutant Pestiviruses, and vaccines containing said viruses. Pestiviruses cause economically important diseases in animals worldwide. The genus Pestivirus, within the family Flaviviridae, comprises at least three species: bovine viral diarrhea virus (BVDV), classical swine fever virus (CSFV), and ovine border disease virus (BDV). The presence of a fourth separate group of Pestiviruses comprising isolates from cattle and sheep has been described, and it is now generally accepted to refer to this additional species as BVDV-2; consequently, classical BVDV strains are named BVDV-1.

CSFV causes classical swine fever in pigs. Classical swine fever is a highly contagious and sometimes fatal disease in pigs, and can cause considerable economic losses.

Animals may be protected against CSFV by vaccination, however, conventional inactivated or modified live vaccines have disadvantages concerning safety as well as efficacy. Therefore, new, improved, types of vaccines should be developed.

Bovine Viral Diarrhea virus (BVDV) is a cause of congenital and enteric disease with a wide range of clinical manifestations. The disease was first described as a transmissible disease of cattle with a high morbidity and low mortality. Affected cattle displayed elevated temperature, diarrhea, and coughing. The condition was termed bovine viral diarrhea. Today, BVDV is considered to be a major pathogen of cattle with a world-wide economic impact. The clinical pattern of BVDV infection within a herd and consequently its impact on production depend on the interaction of several factors, including the strain of the virus, age of the cattle, immunity in the herd, and interplay of stressors. BVDV-1 and BVDV-2 both cause acute infections in cattle (diarrhea, fever, hemorrhagic syndrome) as well as (if the infection occurs during pregnancy) abortion, malformation of the fetus and persistent infection of the calves.

The genome of Pestiviruses consists of a single strand of (+)RNA and contains a single large open reading frame (ORF), flanked by a 5' UTR and a 3' UTR. The ORF encodes a large precursor polyprotein which gives rise to 11 to 12 cleavage products. The ORF is processed into the various viral proteins by cellular- as well as viral proteases. The viral proteins in the ORF are arranged in the order: NH₂-Npro-C-Erns-E1-E2-p7-NS2-NS3-NS4A-NS4B-NS5A-NS5B-COOH. Pestivirus virions consist of four structural proteins, the small positively charged Core (C) protein, that is supposed to form together with the RNA genome a nucleocapsid structure, and three glycosylated envelope proteins (Erns, E1, E2). Neutralizing activity was predominantly demonstrated for E2-specific antibodies.

The minimal requirements for Pestivirus replication were investigated, for example, by creating defective Pestivirus genomes lacking the gene sequences for the structural proteins. It was found that the defective CSFV genomes still replicated and could be packaged into viral particles when introduced in SK-6 cells together with helper A187-CAT RNA (Moser et al., J.Virol., 7787-7794, 1999). An autonomously replicating, but defective, BVDV genome which lacks the genes encoding C, Ems, E1, E2, p7 and NS2 had been described (Behrens et al., J.Virol., 72, 2364-2372, 1998). In trans complemented C and E1 deletion mutants of another pestivirus, BVDV, were described by Beer et al., in WO04/016794.

For CSFV, mutants have been disclosed that show viral attenuation. For example, Risatti et al., Virology 364, 371-382, 2007, describe CSFV mutants with substitutions in the E2 region which showed an attenuated phenotype. Maurer et al., vaccine, 23(25), 3318-28, 2005 also describe CSFV E2 mutants, lacking all or part of the E2 gene which showed partial protection against lethal challenge with highly virulent CSFV. Meyers et al., Journal of Virology, 73(12), 10224-10235, 1999, describe CSFV mutants with mutations in the gene encoding the Ems protein that lead to mutations. In trans complemented Ems deletion mutants of CSFV were described by Wildjojoatmodjo et al., J. Virol., 74(7), 2973-80, 2000.

The present invention provides Mutant Pestiviruses that have a deletion in the gene encoding the Core protein, resulting in the virus being unable to express a functional Core protein, wherein the virus is further characterised in that it contains one or more mutations in the C-terminal domain of the helicase domain of NS3 that compensate for the lack of a functional core protein.

Preferably, the mutant Pestivirus according to the present invention is a Classical Swine Fever virus (CSFV) or a Bovine Viral Diarrhea virus (BVDV).

The mutation in the gene region encoding the Core protein is such that no functional Core protein can be expressed. This can be achieved by deleting the gene encoding the Core protein in whole or in part.

The signal sequence at the C terminus of the Core protein is essential for a further processing of the polyprotein downstream. In deleting (part of) the Core gene care should therefore be taken that the translocation signal sequences for Ems are retained.

The translocation sequence is a stretch of 18 amino acids located between amino acids 250 and 267, preceding the cleavage site before the N terminus of the CSFV Ems protein (Ala-267/Glu-268). (Rümenapf et al., J. Virol., 65(2), 589-597, 1991; Rümenapf et al., J. Virol., 67(6), 3288-3294, 1993). For BVDV, the cleavage site before the N terminus of the Ems protein is Gly-270/Glu-271.

The exact length of the sequences necessary for downstream processing may vary per strain or cell type. For example, for CSFV the Core coding sequence may be deleted from amino acid 169-248 (p619), thereby fusing the C-terminus of Npro to the C-terminal amino acids of Core protein that constitutes the translocation signal for Ems
(CPLWVTSC₁₆₈/LEKALLAWAVITILLYQPVAA₂₆₇/ENIT).
For BVDV the Core coding sequence may be deleted from amino acid 169-251, thereby fusing the C-terminus of Npro to the C-tenninal amino acids of Core protein that constitutes the translocation signal for BVDV Ems (CPLWVSSC₁₆₈/LEKALLAWAIIALVFFQVTMG₂₇₀/ENIT).
The deletion of most the Core gene allowed replication of the RNA after transfection but no infectious virus progeny was produced.

With the present invention it has been found that the absence of a gene encoding a functional Core protein can be compensated by mutations in the NS3 region of the viral genome.

Introduction of these mutations into a cDNA copy of Pestiviruses in which the Core gene was deleted, (as described above) allowed recovery of infectious viruses.

The mutations in the NS3 region cluster in the C-terminal domain of the helicase domain of NS3, more specifically within 100 amino acids of the C-terminal domain.

For CSFV, seven independent mutations in the of the C-terminal domain of the helicase domain of the NS3 region (amino acids 2160 - 2260) region were identified.

Preferred mutant CSF viruses according to the invention have one or more mutations in the C-terminal domain of the helicase domain of NS3, selected from the group consisting of Asn₂₁₇₇Tyr, Glu₂₁₆₀Gly, Pro₂₁₈₅Thr/Ala, Gln₂₁₈₉Lys, Pro₂₂₀₀Thr, and Asn₂₂₅₆Asp.

Introduction of mutation Asn₂₁₇₇Tyr into CSFV Core Δ (p619) and electroporation of the cRNA led to the surprising release of viable infectious virus particles. The genomic RNA appeared slightly reduced in size in Northern blot analysis and concentrated virus particles did not contain Core protein. Virus titers reached up to 5x10⁵ ffu/ml in SK6 cells and thus were about 10 fold lower than p447 wt titers. Next the other mutations Glu₂₁₆₀Gly, Pro₂₁₈₅Ala, Gln₂₁₈₉Lys, Pro₂₂₀₀Thr, and Asn₂₂₅₆Asp were introduced into CSFV Core Δ (p619) giving rise to p1033 (Ala₂₁₈₅), p1034 (Thr₂₂₀₀), p1035 (Lys₂₁₈₉), p1036 (Asp₂₂₅₆), p1037 (Gly₂₁₆₀) and p1038 (Asp₂₂₅₆). In all cases virus was recovered after electroporation of the respective SP6 transcripts but the viability differed significantly. (growth curve analysis). Most viable was the genome containing the Asn₂₁₇₇Tyr exchange.

Most preferred is thus a mutant CSFV according to the invention, wherein the mutation in the C-terminal domain of the helicase domain of NS3 comprises the Asn₂₁₇₇Tyr mutation.

To study whether these mutations have an additive effect the strongest compensating mutants were combined. For double mutations Asn₂₁₇₇Tyr was combined with Glu₂₁₆₀Gly, Pro₂₁₈₅Ala, Gln₂₁₈₉Lys, Pro₂₂₀₀Thr, and Asn₂₂₅₆Asp. Triple mutations contained Asn₂₁₇₇Tyr, together with Pro₂₁₈₅Ala and Pro₂₂₀₀Thr, Pro₂₁₈₅Ala and Gln₂₁₈₉Lys, Gln₂₁₈₉Lys and Pro₂₂₀₀Thr and Glu₂₁₆₀Gly and Gln₂₁₈₉Lys. All tested combinations showed a reduced viability compared to the single mutations indicating that the addition of enabling mutation improves NS3 in the compensation of Core.

While no physical or phenotypical differences to the parental CSFV wt were apparent, CSFV Core Δ was highly attenuated in the natural host. The mutant viruses according to the invention are therefore suitable for use in vaccines to protect swine against infection with CSFV.

For BVDV, eight independent mutations in the C-terminal domain of the helicase domain of the NS3 region (amino acids 2169 - 2269) region were identified.

Preferred mutant BVD viruses according to the invention have one or more mutations in the C-terminal domain of the helicase domain of NS3, selected from the group consisting of Glu₂₁₈₉Lys, Leu₂₁₉₀Pro, Thr₂₁₉₁Ala, Asp₂₁₉₂Glu, Pro₂₁₉₄Leu, Tyr₂₂₀₄His, Asn₂₂₆₅Tyr, Asn₂₂₆₅Asp.

Most preferred is a mutant BVDV according to the invention wherein the mutation in the C-terminal domain of the helicase domain of NS3 comprises the Asp₂₁₉₂Glu, Tyr₂₂₀₄His, Asn₂₂₆₅Tyr, or Asn₂₂₆₅Asp mutation.

Core protein is apparently not essential for the formation of infectious particles. Apparently C is not required for pestivirus assembly but provides other functions. Its function is compensated by mutations in the helicase domain of NS3. The exact sequence of the residual amino acids of Core protein covering the translocation signal (LEKALLAWAVITILLYQPVAA₂₆₈) for CSFV Erns or (LEKALLAWAIIALVFFQVTMG₂₇₀) for BVDV Erns (the sequence spanning the distance between the C - terminus ofNpro and the N-end of Ems) is not required for formation of infectious virus particles as long as translocation of Ems is maintained. The signal sequence of an unrelated glycoprotein can functionally replace the authentic sequence. A construct in which the residual amino acids of Core has been entirely replaced by a N-terminal CD46 translocation signal is viable (CD46 just serves as an example for a translocated cellular protein). When the transmembrane domain of bovine CD46 (SSGRSPGWLLLAPLLLLPTSSDA) was introduced, viable virus was recovered reaching titers of 2 x10⁴ ffu/ml.

Propagation of Pestiviruses in the absence of Core opens the opportunity to a Pestivirus marker vaccine. It can be expected that antibodies directed against Core protein are present in the sera of animals that have overcome an infection with Pestivirus. Core is immunogenic and expressed in significant amounts.

A vaccine containing infectious viral particles according to the invention together with a pharmaceutically acceptable carrier is likewise part of the present invention.

### EXAMPLES:

### Example 1: Core deletion constructs

To test the hypothesis that the substitutions in NS3 compensate for a disabled Core protein the nucleotide changes were introduced into an infectious CSFV cDNA in which the Core coding sequence (aa169 - 248 (SDDGASG - KPPESRKKL) was deleted (p619). Thus the C-terminus of Npro is fused to the C-terminal amino acids of Core that constitutes the translocation signal for Erns (CPLWVTSC₁₆₈/LEKALLAWAVITILLYQPVAA₂₆₇/ENIT). The deletion of most the Core gene allowed replication of the RNA after transfection but no infectious virus progeny was produced. Introduction of mutation Asn₂₁₇₇Tyr into CSFV Core Δ and electroporation of the cRNA led to the surprising release of viable infectious virus particles. The genomic RNA appeared slightly reduced in size in Northern blot analysis and concentrated virus particles did not contain Core protein. Virus titers reached up to 5x10⁵ ffu/ml in SK6 cells and thus were about 10 fold lower than p447 wt titers. Next the other mutations Gl_{U2160}Gly, Pro₂₁₈₅Ala, Gln₂₁₈₉Lys, Pro₂₂₀₀Thr, and Asn₂₂₅₆Asp were introduced into CSFV Core Δ (p619) giving rise to p1033 (Ala₂₁₈₅), p1034 (Thr₂₂₀₀), p1035 (Lys₂₁₈₉), p1036 (Asp₂₂₅₆), p1037 (Gly₂₁₆₀) and p1038 (Asp₂₂₅₆). In all cases virus was recovered after electroporation of the respective SP6 transcripts but the viability differed significantly. Most viable was the genome containing the Asn₂₁₇₇Tyr exchange (mutant "CSFV1017 Core Δ"). Core protein is apparently not essential for the formation of infectious particles. Its function is compensated by mutations in the helicase domain of NS3.

To assess whether the compensation of an absent Core by mutations in NS3 also applies to BVDV the Core coding sequence of BVDV cDNA clone NCP7 (GeneBank accession number AF220247) was removed by PCR on a subclone containing the XhoI/BglII fragment (nt 222 - 2335) using primers BVT86 (GCAGCTTGAAACCCATAGGGGGCAG) and Core268 (CTAGAGAAAGCCCTATTGGCCTG). The deletion was verified by sequencing and introduced into the full length cDNA clone by ligation of a XhoI/NcoI fragment (nt 222 - 4671). The resulting full length plasmid lacking Core (p1026) encoded the N^{pro} preceeding the signal sequence fo E^{rns} analogous to CSFV Core Δ (p619). Transcripts of p1026 were replicative in MDBK cells but no infectious virus was observed. Introduction of substitutions Pro₂₁₉₄Ala, Pro₂₂₀₉Tyr and Asn₂₂₆₅Asp into NCP7ΔC allowed the rescue of infectious virus titers of about 10³ ffu/ml when titered on MDBK cells. A modified NCP7ΔC that was engineered for propagation in porcine SK6 cells revealed Core compensating at Tyr₂₂₀₄His, Asn₂₂₆₅Tyr, Asn₂₂₆₅Asp, Pro₂₁₉₄Leu, Gln₂₁₉₈Lys and Leu₂₁₉₉Pro. NCP7 lacking Core protein virus was rescued from SK6 cells with significant titers (Asn₂₂₆₅Tyr: 3.3x10⁵, Tyr₂₂₀₄His: 2x10⁴ and Asp₂₁₉₂Glu: 1.5x10⁴ ffu/ml).

On the basis of an infectious copy of BVDV C86, a regular Type1 BVDV virus that grows well in MDBK cells, a Core deletion mutant was generated as described above and the mutation analogous to Asn2265Asp (Asn2433Asp) was introduced. Virus with a titer of 5x10⁴ ffu was recovered after transfection in SK6 cells.

### Core protein derived transmembrane domain can be functionally replaced by a host cellular sequence

To exclude a functional importance of the residual amino acids covering the translocation signal (LEKALLAWAVITILLYQPVAA₂₆₈) for Ems the sequence spanning the distance between the C - terminus ofNpro and the N-end of Ems was replaced in CSFV Core Δ _{N2177Y} (p1017) by the transmembrane domain of bovine CD46 (SSGRSPGWLLLAPLLLLPTSSDA). SP6 Transcripts of the respective cDNA (p1246) were transfected into SK6 cells. Viable virus was recovered reaching titers of 2 x10⁴ ffu/ml.

### Material and methods

### Cells and Viruses

SK6 cells were propagated on plastic dishes in DMEM containing 10% Fetal calf serum and non essential amino acids in a incubator with 5% CO2. Cells were split every three days. All CSFV viruses are derived from non-cp CSFV strain Alfort-p447 (Gallei et al., J. Virol., 79(4), 2440-2448, 2005).

### Electroporation:

2µg of SP6 Transcript were electroporated into 5x106 SK6 cells. For this purpose SK6 cells (90% confluency) were trypsinized, washed twice in PBS (Ca++ and Mg++) mixed with the transcript in a total volume of 310µl and subjected to electroporation (Biorad Genepulser, 0.18KV and 0.95µFd) in cuvettes with a 2mm gap. Cells were recovered from the cuvette and plated on 35mm plastic dishes in DMEM supplemented with 10% fetal calf serum.:

### RNA preparation and cDNA synthesis:

SK6 cells infected with rescued virus were washed and the RNA prepared using Rneasy Kit (Quiagen) as recommended by the manufacturer. 2µg of RNA were subjected to reverse transcription using different primers depending on the actual location of interest. PCR was performed with suitable primers and sub-cloned into pGEM-T vector (Promega).

### Example 2: Animal Experiment.

An animal experiment was carried out in order to characterize the virulence of Classical Swine Fever Virus (CSFV) mutant "CSFV1017 Core Δ" under experimental conditions.

### Objective:

The main objective of this experiment was to investigate whether CSFV mutant "CSFV 1017 Core Δ" shows attenuation in comparison with the parental strain "CSFV Alfort p447". The use of sentinel pigs allowed to roughly investigate shedding and transmission of the virus.

As attenuation was proven, the remaining pigs were challenged on January 2^{nd} with the highly pathogenic CSFV strain Koslov.

### Animal experiment:

### Part 1 - Infection

Six weaner pigs were bought from a commercial pig farm on October 16^{th} 2006 and brought into the high containment unit of the Institute of Virology. Animals were randomly divided into two groups of three pigs each. All animals were individually marked with numbers and ear tags.

The two groups (group 1: animals with numbers 75-77; group 2: animals with numbers 78-80) were kept under high containment conditions (negative air pressure of 100 Pa) in separate stables without any contact to each other throughout the experiment. Animals were fed once a day. Water was supplied *ad libitum.* Animals were clinically healthy at the beginning of the trial.

Prior to the beginning of the experiment blood samples were collected from all animals and investigated for the presence of neutralizing antibodies to Pestiviruses (CSFV, Border Disease Virus (BDV) and Bovine Viral Diarrhea Virus (BVDV) by neutralization assays.

After a period of adaptation to the new environment, two pigs of each group were infected intramuscularly with app. 10⁷TCID₅₀ of CSFV₁₀₁₇ (group 1) and CSFV Alfort p447 (group 2), respectively. Sentinel pigs (one per group) were removed from the groups for at least 12 h post inoculation. After this period they were put to the respective groups again.

Animals of both groups were monitored daily for clinical symptoms and body temperature. Body temperatures exceeding 40°C were recorded as fever. During the course of the experiment, animals showing clinical symptoms which were no longer tolerable for animal welfare reasons were euthanized. Post-mortem examinations of all animals were performed and recorded.

Blood samples (native blood and EDTA anticoagulated blood) were taken from all animals on days 0, 2, 4, 7, 10, 14, 21, and 28 post infection. Additional samples were taken on the day of euthanasia. Native blood samples were centrifuged at 1500 rpm for 15 min at room temperature (Labofuge 400R, Heraeus, Germany) to obtain serum. Serum was then divided into suitable aliquots of 0.5 to 2 ml and stored at -80°C until further testing.

### Part II - Challenge

Pigs remaining after the infection part (Part I) were intranasally challenged on (71^{st} day post infection) with 10^{4.5}TCID₅₀ of the highly pathogenic CSFV strain Koslov. Blood samples were taken on days 0, 3, 6 post challenge and on the day of euthanasia of the respective pig.

### Laboratory examinations

### Leukocyte counts

Leukocytes were counted from EDTA blood manually using the counting chamber according to Neubauer (improved) after erythrocyte lysis with acetic acid (2%). EDTA blood was drawn into a blood diluting pipette according to Thoma up to the 0.5 mark. Then, acetic acid (2%) was added up to the 11 mark and the sample dilution was shaken gently for app. 60 sec. After filling the dilution into a prepared counting chamber, leukocytes were counted in the leukocyte counting fields under light microscope. The correct value was calculated according to chamber specifications. Values below 10 G/l were recorded as leucopenia.

Leukocyte counts were performed on days 0, 2, 4, 7, 10, 14, 21, and 28 post infection.

### Neutralization peroxidase-linked antibody assay

Neutralization peroxidase-linked antibody assays (NPLA) were performed according to the "Diagnostic Manual" of the European Commission (Commission Decision 2002/106 of Feb. 1^{st}, 2002) and the Technical Part accompanying it, utilizing the following viruses for the detection of antibodies against CSFV and BVDV/Pestiviruses, respectively:
- BVDV NADL
- CSFV reference strain "Alfort 187" (CSF 902)
- CSFV Alfort p447 (the parental strain)
- CSFV Koslov (challenge strain)
- CSFV Diepholz (CSF0104)

NPLAs for the detection of BVDV antibodies were carried out on primary fetal calve kidney cells, whereas NPLAs for the detection of CSFV antibodies were carried out on permanent porcine kidney cells (SK 6). Antibody titers are expressed as the reciprocal of the highest initial serum dilution which prevented virus replication in 50% of two replicate wells. These neutralization dilutions 50% (ND₅₀) were calculated using the method of KÄRBER.

NPLAs for the detection of CSFV antibodies were carried out on days 0, 10, 14, 21, and 28 days post infection (CSFV Alfort p447, and CSF0902). Additionally, samples taken on days 0, 3, and 6 post challenge, and on the days of euthanasia were investigated (CSFV Alfort p447, and CSF0902). Samples taken post challenge were subjected to a NPLA using CSFV Koslov and CSF0104 as well. NPLAs for the detection of neutralizing antibodies against BVDV were performed prior to the beginning of the experiment.

### Antibody enzyme-linked immunosorbent assay (ELISA)

In addition to the NPLAs, serum samples collected on days 0, 14, 21, and 28 post infection and from the days of euthanasia were subjected to a commercially available antibody enzyme-linked immunosorbent assay (ELISA) for the detection of antibodies directed against CSFV. All tests were performed exactly as described in the manufacturers' instructions.

The following ELISA test kit was used:
- HerdChek CSFV, Idexx Laboratories, Wörrstadt, Germany

### Virus isolation

Virus isolation from leukocyte preparations was performed over two passages on permanent porcine kidney cell lines SK6 and Rie 5-1 according to the EU Diagnostic Manual (Commission Decision 2002/106/EC). Indirect immune labeling was done with the pestivirus specific monoclonal antibody BVD/C16 (Peters et al., 1986) and a commercial species specific peroxidase-linked conjugate (RAMPO, Dako).

### Antigen ELISA

In addition to virus isolation, a commercially available antigen enzyme-linked immunosorbent assay (ELISA) was performed exactly as described in the manufacturers' instructions. Samples taken on days 0, 4, 7, and 10 were subjected to analyses.

The following ELISA test kit was used:
- CHEKIT HCV-Antigen, former Dr. Bommeli AG, Liebefeld-Bern, Switzerland

### Results

### Part I - Infection

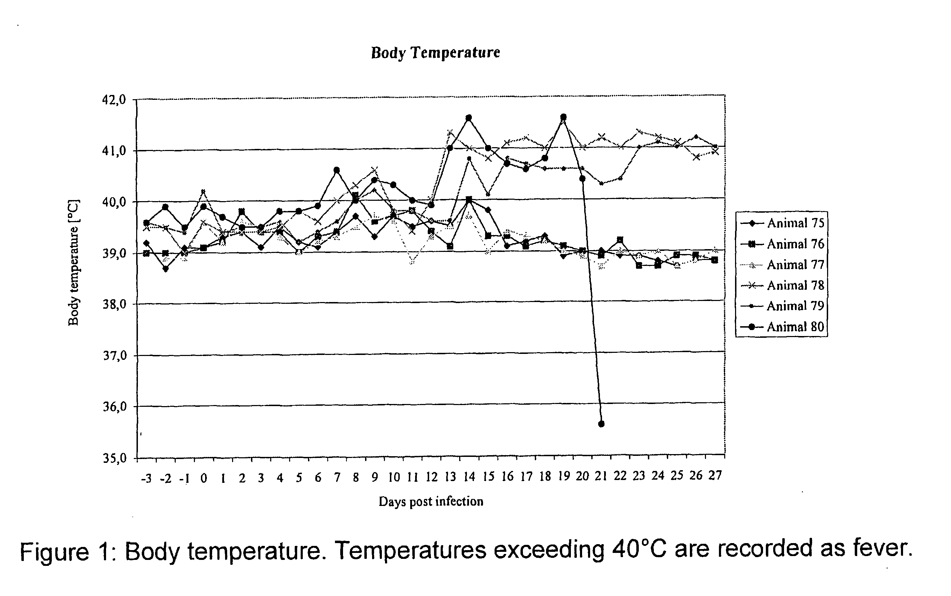

### Clinical signs and temperature (see figure 1)

During the infection part of the experiment, only animal 76 showed a slight fever on day eight post infection (40.1°C). Sub febrile temperatures of 40.0°C were recorded for animals 75 and 76 on day 14 post infection.

In contrast, all animals of group 2 developed fever. Animal 78 showed febrile temperatures on days eight and nine post infection, and from day 13 post infection till the end of the experiment on day 28. Animal 79 showed fever on day 9 post infection, and from day 14 till the end of the experiment. Febrile temperatures were recorded for animal 80 on days seven, nine, ten, and from day 13 till day 20 post infection. On day 21, animal 80 showed a body temperature of 35.6°C.

While animals of group 1 (75-77) did not develop any clinical signs indicative for a CSFV infection during the first part of the experiment, all animals of group 2 (78-80) developed distinct clinical symptoms. The severity of clinical signs varied among the animals. Animal 76 was euthanized unrelated to CSF infection on day 44 post infection due to severe lameness.

During the course of the experiment, animal 78 developed severe diarrhea, conjunctivitis, anorexia, skin lesions, and ataxia. The animal was euthanized on day 28 post infection. Animal 79 developed only slight, uncharacteristic symptoms including reduced appetite, and slight diarrhea. The animal was euthanized on day 28 post infection. Animal 80 developed severe symptoms including gastrointestinal and respiratory signs, anorexia, dullness, and skin lesions. The animal was euthanized moribund on day 21 post infection.

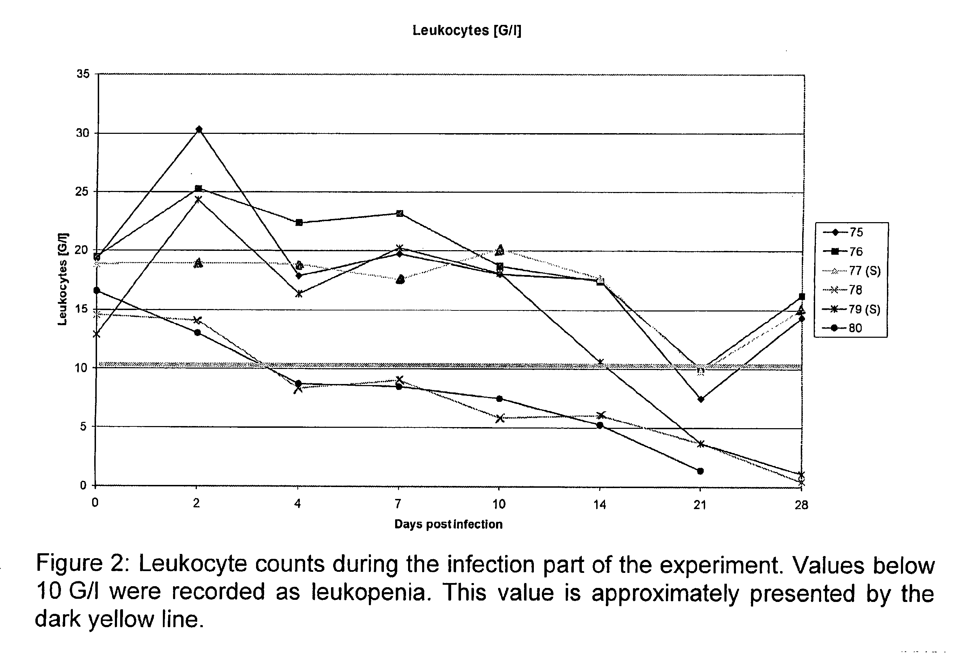

### Leukocyte counts (figure 2)

In general, animals of group 1 did not show leucopenia. Nevertheless, animals 75 and 77 showed values below 10 G/l on day 20 post infection. On the same day, animal 76 showed only 10.3 G/l.

All animals of group 2 developed a severe leucopenia upon infection with CSFV Alfort p447. In animal 78, leucopenia started on day 4 post infection, and only 0.45 G/l were recorded on the day of euthanasia. The sentinel pig 79 showed leukopenic values on days 21 and 28 post infection, and a limit value of 10.6 G/l already on day 14 post infection. Animal 80 showed severe leucopenia starting on day 4 post infection. The terminal value was 1.4 G/l on day 21 post infection.

### Pathology

Animals of group 1 were not euthanized during the infection part of the experiment.

Animals of group 2 showed varying pathological signs. Animal 78 showed diffuse dermatitis, swollen and Haemorrhagic lymph nodes in the gut and respiratory tract, thymus atrophy, bronchopneumonia, ulcerative gastritis, catarrhal enteritis, pyelonephiritis, and petechial bleedings in cortex of the kidneys. Animal 79 showed a slight thymus atrophy, slightly swollen lymph nodes in the gut and the respiratory tract, pneumonia (cranial lobes), ulcerative gastritis, severe catarrhal enteritis, and cystitis. Animal 80 showed petechial bleedings of the cutis and subcutis (acra, abdomen), profoundly enlarged, oedematous and Haemorrhagic lymph nodes (systemic), severe thymus atrophy, petechial bleedings in the meninges, spleen, epiglottis, trachea, stomach, gut wall, ileocaecal valve, bladder, liver, gall bladder, and the kidneys. In addition, animal 80 showed a catarrhal-purulent pneumonia, extensive hemorrhages beneath the endocardium and within the heart muscle, severe Haemorrhagic gastritis, constipation, and a severe intestinal nephritis.

### Antibody Detection

### Neutralization peroxidase-linked antibody assay (NPLA)

All animals were tested negative for BVDV/Pestivirus antibodies prior to the experiment. All samples taken on day 0 showed negative (<5 ND₅₀) results with both CSFV strains used in the NPLA (CSF0902 and Alfort p447).

Using CSFV strain Alfort p447, animals of group 1 showed positive results starting on day 14 post infection (animal 75). In detail, animal 75 showed an antibody titre of 7.5 ND₅₀ on day 14 post infection which increased thereafter to 316 ND₅₀. Animal 76 showed positive results on days 21 and 28 post infection reaching an antibody titre of 158,5 ND₅₀. The sentinel, animal 77, did not develop any antibody titre.

Using CSF0902, only animal 76 showed a low antibody titre of 7.5 ND₅₀ on day 28 post infection. Animals of group 2 remained negative (<5 ND₅₀) for CSFV antibodies in all NPLAs performed throughout the experiment.

### Antibody enzyme-linked immunosorbent assay (Ab-ELISA)

While animal 75 showed a first doubtful result on day 21 post infection and was positive on day 28 post infection, animal 76 showed a doubtful result on day 14 post infection and positive results on days 21 and 28 post infection. Animal 77 remained negative.

Animals 78 and 79 showed negative results throughout the trial whereas animal 80 showed a doubtful result on the day of euthanasia (21 days post infection).

### Virus isolation

Animals of group 1 showed negative results throughout the infection part of the trial on both SK6 and Rie 5-1 cells.

Animal 78 showed positive results on days four, seven, ten (investigated on SK6 cells only), and 14 post infection on SK6 and Rie 5-1 cells, respectively. In addition, a positive result was achieved for this pig on day two post infection on Rie 5-1 cells. The sentinel pig, animal 79, remained negative on both cell lines. On SK 6 cells, animal 80 showed positive results on days seven, ten, and 14 post infection. Positive results were also found on Rie 5-1 cell on days seven and 14 (day ten not tested due to lack of sample material).

### Antigen enzyme-linked immunosorbent assay (Ag-ELISA)

The Ag-ELISA remained negative for all animals of group 1, and for animals 78 and 79 of group 2. Only animal 80 showed a positive result on day 10 post infection.

### Part II - Challenge

### Clinical signs and body temperature

Pigs remaining after the infection part, animals 75 and 77, were challenged with the highly pathogenic CSFV strain Koslov on day 71 post infection.

Animal 75 did not develop febrile temperatures or any clinical signs upon challenge infection and was euthanized on day 16 post challenge.

Animal 77 developed fever on day three post challenge that persisted till euthanasia. The animal was euthanized moribund on day eight post challenge showing severe neurological symptoms.

### Pathology

Prior to the challenge, animal 76 was euthanized due to severe lameness. This animal did not show any pathological lesions indicative for CSF. It only showed a slight pneumonia of the cranial lobes. Beside a slight pneumonia of the cranial lobes and a slight ulcerative gastritis, animal 75 did not show pathological signs. Animal 77 showed swollen, partly Haemorrhagic lymph nodes, catarrhal pneumonia, ascites, slight ulcerative gastritis, liver congestions, gall bladder edema, and intestinal nephritis.

### Antibody Detection

After challenge infection, animal 75 showed an antibody titre against the challenge virus of 160 ND₅₀ (day 16 post challenge), and a titre of >640 against CSF0104. Animal 77 remained negative.

### Summary and Conclusions

- CSFV mutant "CSFV 1017 Core Δ" showed virtually full attenuation in comparison with the parental strain "CSFV Alfort p447"
- In contrast to animals of group 2, no virus could be detected in virus isolation on different cell lines for animals of group 1.
- Except for the sentinel pig, animals of group 1 developed neutralizing antibodies upon infection with CSFV 1017 Core Δ
- The sentinel pig of group 1 did not show any positive results in both antibody and antigen detection. Thus, shedding and transmission of the virus mutant is most unlikely.
- The absence of clinical signs upon challenge infection with a highly pathogenic CSFV strain proved full protection of the animal previously infected with CSFV 1017 Core Δ
- The sentinel pig showed an acute-lethal CSF course upon challenge infection with CSFV Koslov

## Claims

1. Mutant Pestivirus, **characterised in that** the virus has a mutation in the gene encoding the Core protein, resulting in the virus being unable to express a functional Core protein, the virus further being **characterised in that** it contains one or more mutations in the C-terminal domain of the helicase domain of NS3 that compensate for the lack of a functional core protein.

2. Mutant Pestivirus according to claim 1, **characterised in that** the Pestivirus is Classical Swine Fever Virus (CSFV).

3. Mutant CSFV according to claim 2, **characterised in that** the mutation in the C-terminal domain of the helicase domain of NS3 is selected from the group consisting of Asn₂₁₇₇Tyr, Glu₂₁₆₀Gly, Pro₂₁₈₅Thr/Ala, Gln₂₁₈₉Lys, Pro₂₂₀₀Thr and Asn₂₂₅₆Asp.

4. Mutant CSFV according to claim 2 or 3, **characterised in that** the mutation in the C-terminal domain of the helicase domain of NS3 comprises the Asn₂₁₇₇Tyr mutation.

5. Mutant Pestivirus according to claim 1, **characterised in that** the Pestivirus is Bovine Viral Diarrhea virus (BVDV).

6. Mutant BVDV according to claim 5, **characterised in that** the mutation in the C-terminal domain of the helicase domain of NS3 is selected from the group consisting of Glu₂₁₈₉Lys, Leu₂₁₉₀Pro, Thr₂₁₉₁Ala, Asp₂₁₉₂Glu, Pro₂₁₉₄Leu, Tyr₂₂₀₄His, Asn₂₂₆₅Tyr, Asn₂₂₆₅Asp.

7. Mutant BVDV according to claim 5 or 6, **characterised in that** the mutation in the C-terminal domain of the helicase domain of NS3 comprises the Asp₂₁₉₂Glu, Tyr₂₂₀₄His, Asn₂₂₆₅Tyr, or Asn₂₂₆₅Asp mutation.

8. A vaccine **characterised in that** it comprises a mutant Pestivirus according to any of the preceding claims and a pharmaceutically acceptable carrier.

## Patentansprüche

1. Mutiertes Pestivirus, **dadurch gekennzeichnet, dass** das Virus eine Mutation in dem für das -Core-Protein codierenden Gen aufweist, wodurch das Virus nicht in der Lage ist, ein funktionsfähiges Core-Protein zu exprimieren, wobei das Virus ferner **dadurch gekennzeichnet ist, dass** es eine oder mehrere Mutationen in der C-terminalen Domäne der Helicase-Domäne von NS3 enthält, die das Fehlen eines funktionsfähigen Core-Proteins ausgleichen.

2. Mutiertes Pestivirus nach Anspruch 1, **dadurch gekennzeichnet, dass** es sich bei dem Pestivirus um CSFV (Classical Swine Fever Virus) handelt.

3. Mutiertes CSFV nach Anspruch 2, **dadurch gekennzeichnet, dass** die Mutation in der C-terminalen Domäne der Helicase-Domäne von NS3 aus der aus Asn₂₁₇₇Tyr, Glu₂₁₆₀Gly, Pro₂₁₈₅Thr/Ala, Gln₂₁₈₉Lys, Pro₂₂₀₀Thr und Asn₂₂₅₆Asp bestehenden Gruppe ausgewählt ist.

4. Mutiertes CSFV nach Anspruch 2 oder 3, **dadurch gekennzeichnet, dass** die Mutation in der C-terminalen Domäne der Helicase-Domäne von NS3 die Asn₂₁₇₇Tyr-Mutation umfasst.

5. Mutiertes Pestivirus nach Anspruch 1, **dadurch gekennzeichnet, dass** es sich bei dem Pestivirus um BVDV (Bovine Viral Diarrhea Virus) handelt.

6. Mutiertes BVDV nach Anspruch 5, **dadurch gekennzeichnet, dass** die Mutation in der C-terminalen Domäne der Helicase-Domäne von NS3 aus der aus Glu₂₁₈₉Lys, Leu₂₁₉₀Pro, Thr₂₁₉₁Ala, Asp₂₁₉₂Glu, Pro₂₁₉₄Leu, Tyr₂₂₀₄His, Asn₂₂₆₅Tyr, Asn₂₂₆₅Asp bestehenden Gruppe ausgewählt ist.

7. Mutiertes BVDV nach Anspruch 5 oder 6, **dadurch gekennzeichnet, dass** die Mutation in der C-terminalen Domäne der Helicase-Domäne von NS3 die Asp₂₁₉₂Glu-, Tyr₂₂₀₄His-, Asn₂₂₆₅Tyr- oder Asn₂₂₆₅Asp-Mutation umfasst.

8. Impfstoff, **dadurch gekennzeichnet, dass** er ein mutiertes Pestivirus nach einem der vorhergehenden Ansprüche sowie einen pharmazeutisch unbedenklichen Trägerstoff umfasst.

## Revendications

1. Pestivirus mutant, **caractérisé en ce que** le virus a une mutation dans le gène codant pour la core protéine, avec pour conséquence que le virus devient incapable d'exprimer une core protéine fonctionnelle, le virus étant en outre **caractérisé en ce qu'**il contient une ou plusieurs mutations dans le domaine C-terminal du domaine hélicase de NS3, qui compensent l'absence de core protéine fonctionnelle.

2. Pestivirus mutant selon la revendication 1, **caractérisé en ce que** le pestivirus est le virus de la peste porcine classique (CSFV).

3. CSFV mutant selon la revendication 2, **caractérisé en ce que** la mutation dans le domaine C-terminal du domaine hélicase de NS3 est choisie dans le groupe consistant en Asn₂₁₇₇Tyr, Glu₂₁₆₀Gly, Pro₂₁₈₅Thr/Ala, Gln₂₁₈₉Lys, Pro₂₂₀₀Thr et Asn₂₂₅₆Asp.

4. CSFV mutant selon la revendication 2 ou 3, **caractérisé en ce que** la mutation dans le domaine C-terminal du domaine hélicase de NS3 comprend la mutation Asn₂₁₇₇Tyr.

5. Pestivirus mutant selon la revendication 1, **caractérisé en ce que** le pestivirus est le virus de la diarrhée virale bovine (BVDV).

6. BVDV mutant selon la revendication 5, **caractérisé en ce que** la mutation dans le domaine C-terminal du domaine hélicase de NS3 est choisi dans le groupe consistant en Glu₂₁₈₉Lys, Leu₂₁₉₀Pro, Thr₂₁₉₁Ala, Asp₂₁₉₂Glu, Pro₂₁₉₄Leu, Tyr₂₂₀₄His, Asn₂₂₆₅Tyr, Asn₂₂₆₅Asp.

7. BVDV mutant selon la revendication 5 ou 6, **caractérisé en ce que** la mutation dans le domaine C-terminal du domaine hélicase de NS3 comprend la mutation Asp₂₁₉₂Glu, Tyr₂₂₀₄His, Asn₂₂₆₅Tyr ou Asn₂₂₆₅Asp.

8. Vaccin, **caractérisé en ce qu'**il comprend un pestivirus mutant selon l'une quelconque - des revendications précédentes et un support pharmaceutiquement acceptable.
